# EUROPEAN PATENT APPLICATION

(11) **EP 4 729 883 A1**
(43) Date of publication of application: **22.04.2026**
(21) Application number: 24207520.8
(22) Date of filing: 18.10.2024
(51) Int. Cl.: G01B 11/16, A61B 1/005, G02B 6/02

(54) **MULTICORE OPTICAL FIBER AND ELONGATED DEVICE FOR MEDICAL INTERVENTIONAL APPLICATION**

(71) Applicant: Koninklijke Philips N.V., 5656 AG Eindhoven (NL)
(72) Inventor: MARCELIS, Bout, Eindhoven (NL); DIJKKAMP, Dirk, Eindhoven (NL); GUNDERSON, Richard Charles, 5656AG Eindhoven (NL); VAN DEN ENDE, Daan Anton, Eindhoven (NL)
(74) Representative: Philips Intellectual Property & Standards

(57) **Abstract**

The present invention relates to a multicore optical fiber (10) configured for optical shape sensing. The optical fiber (10) comprises a cladding (12) and a plurality of fiber cores (14, 16, 18, 20) embedded in the cladding (12). The fiber cores (14, 16, 18, 20) each have a reflective strain sensitive structure (21) responsive to strain in the optical fiber (10). The cladding (12) comprises, along a length of the optical fiber (10), a main section (22) and a distal tip section (24) arranged distally from the main section (22). The main section (22) has a first width along a first axis transverse to a longitudinal axis of the optical fiber (10) and a first number of fiber cores, and the tip section (24) has a second width along a second axis parallel to said first axis and a second number of fiber cores. The second width is reduced with respect to the first width such that a bending stiffness of the tip section (24) about a bending axis orthogonal to said second axis is lower than the bending stiffness of the main section (22) about a bending axis orthogonal to said first axis. An elongated device for medical interventional application comprising such a multicore optical fiber is also disclosed.

## Description

### FIELD OF THE INVENTION

The present invention generally relates to the field of optical shape sensing technology, also referred to as Fiber Optic RealShape (FORS) technology. In particular, the present invention relates to a multicore optical fiber and an elongated device for medical interventional application comprising a multicore optical fiber.

### BACKGROUND OF THE INVENTION

Fiber Optic RealShape (FORS) technology is a shape sensing and reconstruction technology used in elongated devices, like guidewires, to enable accurate, three-dimensional and real-time visualization of the devices to allow efficient navigation during minimally invasive endovascular procedures without the need for X-ray guidance. The reduction of X-ray radiation exposure during such a procedure is beneficial for both patients as well as physicians and staff.

The FORS technology uses a shape sensing enabled elongated device, like a guidewire, for use in the procedure and a measurement system, which typically comprises a light source and an optical detector compatible with the shape sensing enabled elongated device, as well as a signal processing unit and a user interface for viewing and tracking the visualized elongated device.

FORS-enabled devices typically include an integrated twisted multicore optical glass fiber with sensors which may be configured as distributed fiber Bragg grating (FBG) sensors. Light from the light source is used to interrogate the sensors and reflections are analyzed to determine the local deformation of each segment of the optical fiber such that the full three-dimensional shape can be reconstructed. In this way, the full shape of elongated devices can be reconstructed and visualized in real time during the procedure. The FORS enabled devices may be visualized in the context of the patient's anatomy through overlay on images acquired before or during the procedure, like CT scans and X-rays. The amount of images are thus significantly reduced compared to X-ray guided procedures where a new X-ray image is needed every time the elongated device needs to be visualized, leading to a marked reduction in X-ray dose.

The applicant of the present application has developed FORS enabled guidewires and catheters, which have been successfully employed in complex aortic interventional procedures, in particular fenestrated endovascular aortic aneurysm repair (FEVAR) and/or branched endovascular aortic aneurysm repair (BEVAR), as well as in endovascular peripheral lesion repair (EVPLR).

In complex aortic procedures the requirements for mechanical properties of the tip section of the typically used guidewires do not restrict the use of brittle glass fiber sensors which extend up to the distal tip of the elongated device, since navigation often is within large vessels (e.g. aorta), where the guidewire is free to navigate inside the vessel and will typically not encounter obstructions.

In other procedures, such as various peripheral procedures, such as treatment of peripheral artery disease (PAD), FORS enabled elongated devices are also beneficial for reducing radiation dose. However, in these types of procedures, vessels are much smaller in diameter, and often the trajectory of navigation is very tortuous, i.e. with many bends, and potentially many bifurcations must be accessed to get to the target location, often involving acute angles through which the elongated device must travel. Moreover, these smaller vessels are much more fragile and may be damaged by the tip of the elongated device. Especially in PAD, angioplasty techniques require crossing a narrowed or occluded section of a blood vessel. For more severe occlusions, crossing a narrowed or occluded section of a blood vessel requires force, and sometimes the tip of the device prolapses, i.e. the device loops and the tip of the device bends back on itself, a phenomenon also called 'knuckling', causing very tight bending in the device tip, i.e. a bending radius < 1 mm. In these cases, the device tip must also be robust enough to withstand this deformation.

For FORS enabled devices, like guidewires, used in peripheral interventional procedures, the tip of the device must be soft and flexible enough to easily navigate through tight bends and acutely angled bifurcations. For example, the tip load of the combination of the inner optical fiber and the outer shell tip of the device must not exceed the limits required for safe navigation, i.e. without damaging the blood vessels, which is around 2-4 gram force for guidewires used in peripheral procedures.

Also during these procedures, the curvature of the optical fiber may become high enough to impede shape sensing or may even become higher than the maximum curvature that the glass fiber can withstand, resulting in failure of the glass material, i.e. breaking or shattering, during use.

In particular, during procedures where a tortuous anatomy must be navigated and/or a narrow lesion or occlusion must be crossed the forces acting on the tip of the device may result in deformations of the device tip which are too extreme for a glass fiber to handle, especially when the device tip is looped or prolapsed. Breaking of the glass fiber will not only impede the use of the FORS technology, but may also result in a patient safety issue if the glass splinters potentially damage the internal structure of the device tip, further altering its mechanical properties.

At the same time, the shape and direction of the tip of the elongated device must still be reconstructed by the FORS technology as accurate as possible even under such challenging circumstances, involving large deformations due to looping and prolapse, resulting in a tight bending radius, such that the need for X-ray guidance can still be avoided.

US 2012/099112 A1, US 11 135 092 B2, and US 2013/41670 A1 disclose optical fibers having part of the cladding of the optical fiber removed along a length of the fiber. However, these optical fibers are not suitable to solve the technical problem described above.

### SUMMARY OF THE INVENTION

It is an object of the present invention to provide a multicore optical fiber configured for optical shape sensing, in particular for use in an elongated device for medical interventional application, which is suitable for procedures where a tortuous anatomy must be navigated and/or a narrow lesion or occlusion must be crossed, while at the same time allowing reconstruction of the shape and direction of the tip region of the optical fiber.

It is a further object of the present invention to provide an elongated device for medical interventional application which is suitable for procedures where a tortuous anatomy must be navigated and/or a narrow lesion or occlusion must be crossed, while at the same time allowing reconstruction of the shape and direction of the tip region of the elongated device by the FORS technology with sufficient accuracy.

In a first aspect of the present invention, a multicore optical fiber configured for optical shape sensing is provided, comprising a cladding and a plurality of fiber cores embedded in the cladding, the fiber cores each have a reflective strain sensitive structure responsive to strain in the optical fiber, wherein the cladding comprises, along a length of the optical fiber, a main section and a distal tip section arranged distally from the main section, the main section having a first width along a first axis transverse to a longitudinal axis of the optical fiber and a first number of fiber cores, and the tip section having a second width along a second axis parallel to said first axis and a second number of fiber cores, wherein the second width is reduced with respect to the first width such that a bending stiffness of the tip section about a bending axis orthogonal to said second axis is lower than the bending stiffness of the main section about a bending axis orthogonal to said first axis.

The present invention is based on the idea to reduce the thickness of the multicore optical fiber in its distal tip section at least along one axis such that the tip section of the multicore optical fiber according to the invention can withstand smaller bending radii, i.e. higher curvatures in comparison with conventional multicore optical fibers. The main section of the multicore optical fiber preferably remains optimized for high quality shape sensing, such as it is provided, for example, by a conventional 125 µm diameter silica glass fiber, as the main section does not experience high deformations during use. On the other hand, the distal tip section of the optical fiber has a reduced bending stiffness and, thus, can bend at least about one axis with smaller bending radius or higher curvature than the main section. The bending stiffness of the distal tip section of the optical fiber may be reduced in comparison to the bending stiffness of the main section in only one axis which may be a preferential bending direction, or the bending stiffness of the tip section may be reduced in comparison to the main section along more than one axis, for example along two perpendicular axes, wherein in this case the width of the distal tip section may be reduced with respect to the width of the main section along more than one axis, e.g. along two perpendicular axes.

The term 'width' used in the present disclosure in the context of the distal tip section of the optical fiber is to be understood as the thickness of the distal tip section, i.e. is used herein as a synonym for 'thickness'. The term 'width' used in the present disclosure in the context of the main section of the optical fiber is to be understood as the diameter of the main section, i.e. is used herein as a synonym for 'diameter'.

The reflective strain sensitive structure inscribed in the fiber cores of the optical fiber may comprise fiber Bragg gratings.

In a further aspect of the present invention, an elongated device for medical interventional application is provided, comprising:
a main section and a distal terminal section together defining a lumen along a length of the elongated device; and
a multicore optical fiber according to the first aspect which is arranged in the lumen, wherein the tip section of the optical fiber is arranged in the distal terminal section of the elongated device.

Optionally, the distal tip section of the optical fiber is free to rotate inside the lumen of the elongated device.

Due to the reduced width of the distal tip section of the multicore optical fiber according to the invention, the elongated device is suitable for procedures where a tortuous anatomy must be navigated and/or a narrow lesion or occlusion must be crossed, while at the same time allowing reconstruction of the shape and direction of the tip region of the elongated device by the FORS technology with sufficient accuracy.

Preferred embodiments of the invention are defined in the dependent claims. It shall be understood that the claimed multicore optical fiber and the claimed elongated device for medical application have similar and/or identical preferred embodiments, in particular as defined in the dependent claims and as disclosed herein.

In an embodiment of the multicore optical fiber according to the invention, a cladding of the tip section may have at least one flattened lateral surface along the length of the tip section.

Flattening at least one lateral surface of the tip section of the multicore optical fiber reduces the bending stiffness of the tip section about at least one bending axis orthogonal to the axis along which the width of the tip section is reduced. Furthermore, flattening a lateral surface of the distal tip section of the multicore optical fiber is advantageous as it may be achieved by simple techniques like laser ablation, milling, grinding, etc.

Preferably, the cladding of the tip section may have a first flattened lateral surface and a second flattened lateral surface along the length of the tip section, wherein the first flattened lateral surface is opposite the second flattened lateral surface.

This embodiment allows for further reducing the thickness and, thus, the bending stiffness of the tip section of the optical fiber to further lower the minimum bending radius or increasing the maximum curvature of the distal tip section without the risk of a breaking or splintering of the distal tip section. Further, this embodiment is advantageous, if the distal tip section shall have preferred bending directions in two opposite bending directions about the same bending axis.

In a further embodiment, the second number fiber cores may be equal to the first number of fiber cores.

This embodiment is advantageous, because the strain sensitivity of the distal tip section of the multicore optical fiber is not reduced in comparison with the strain sensitivity of the main section of the multicore optical fiber despite the reduction of the thickness of the tip section. When reducing the width of the distal tip section along at least one axis, reduction of the width of the distal tip section is performed such that the fiber cores in the distal tip section remain intact in this embodiment.

In another embodiment, the second number of fiber cores is equal to or larger than 1, but smaller than the first number of fiber cores.

In this embodiment, the distal tip section comprises at least one fiber core or even only one fiber core, while the main section comprises a larger number of fiber cores than the tip section of the optical fiber. The advantage of this embodiment is, that the thickness or width of the distal tip section of the multicore optical fiber can be made very small so that only a small number of fiber cores, such as only one fiber core, remain in the distal tip section. Strain sensitivity of the distal tip section is then reduced, but this embodiment allows even smaller bending radii of the distal tip section without the risk of a break or splintering of the distal tip section.

In the context with the previous embodiment, a further embodiment provides that the fiber cores of a first subset of the fiber cores of the main section terminate at or proximally from the beginning of the tip section, and fiber cores of a second subset of the fiber cores of the main section extend into the tip section.

For example, the number of fiber cores in the main section may be 4, with 3 outer fiber cores and 1 central fiber core, while the distal tip section retains only 1 or 2 fiber cores. Or the total number of cores in the main section could be 7, with 3 outer cores in a wider radial distance from the center axis of the optical fiber, 1 central core and 3 fiber cores in between the central and the outer cores. The 3 outer cores may be terminated at the start of the distal tip section, but the 4 remaining cores which extend into the distal tip section allow for full shape 3D reconstruction of the multicore optical fiber.

It is advantageous, if there is no or only little refractive index step at the transition of the fiber cores from the main section to the distal tip section which could give rise to reflections which in turn can deteriorate the strain measurement. In other words, the fiber or the fibers which extend from the main section to the distal tip section preferably are continuous fiber cores.

In a further embodiment, the fiber cores of the first subset of the fiber cores of the main section terminate under an angle such that reflections of light are reduced.

In this embodiment, front surfaces of the fiber cores of the main section which terminate at the start of the distal tip section, i.e. at the distal end of the main section, are not orthogonal with respect to the longitudinal axis of the optical fiber. This is advantageous, because retro-reflections of interrogation light that may be caused at the fiber core/air interface at the distal end of the main section are reduced or even avoided and, thus, the signal-to-noise ratio of the shape sensing measurement is improved.

In a further embodiment, one or more of the fiber cores of the tip section may be arranged eccentrically with respect to a neutral bending plane of the cladding.

This embodiment is advantageous because even if the number of fiber cores is reduced in the distal tip section in comparison with the number of fiber cores in the main section, the one or more fiber cores in the tip section can transfer bending information of the distal tip section due to its or their eccentricity with respect to the neutral bending plane of the tip section cladding.

In a further embodiment, the fiber cores of the main section comprise a central core arranged centrically with respect to a neutral bending plane of the cladding of the main section, wherein a distal portion of the central core extends into the tip section, and wherein the distal portion of the central core is arranged eccentrically with respect to a neutral bending plane of the cladding of the tip section.

The central fiber core in the main section of the optical fiber typically is not sensitive to bending, because it is arranged centrically with respect to the neutral bending plane of the cladding of the main section. If, however, the cladding of the tip section has a reduced width in an asymmetrical manner with respect to the neutral bending plane of the cladding in the main section, the central core is arranged eccentrically with respect to the neutral bending plane of the cladding in the tip section so that even the central core can sense bending strain and, thus, can provide bending information from the tip section.

In further embodiments, the tip section of the optical fiber may have a bending stiffness about the bending axis in the range from 0.05 Nmm² - 0.2 Nmm².

A bending stiffness of the distal tip section in the given range is significantly lower than the bending stiffness of the optical fiber in the main section. For example, a typical optical glass fiber with 100 µm diameter has a bending stiffness of 0.34 Nmm². A reduction of the width of the optical fiber decreases the bending stiffness of the optical fiber. For instance, removal of 20 µm cladding resulting in a 80 µm diameter will reduce the bending stiffness to 0.14 Nmm².

Further, the tip section of the multicore optical fiber may have a length in a range from 1 to 5 cm.

Preferentially, the main section and the tip section are made from a single monolithic fiber.

The monolithic configuration of the main section and the tip section has the advantage that the multicore optical fiber can be made from a single optical fiber, e.g. by post-processing a standard multicore optical fiber, without the need for connecting two fiber parts to one another.

A method of producing a multicore optical fiber according to the invention may have the following configurations:
Providing a standard multicore optical fiber configured for optical shape sensing, and
post-processing a distal tip section of the fiber that may be exposed to high bending curvatures, to reduce a width of the distal tip section along at least one axis.

Optionally, post-processing the distal tip section may include:
Laser ablation of part of the cladding of the tip section such that part of the cladding material is removed.

Optionally, laser ablation may include femtosecond laser ablation.

Femtosecond laser ablation is a technology allowing highly precise removal of materials while minimizing the heat affected zone around the processed area, which is typically less than a few micrometers. Hence, this form of laser ablation is also referred to as 'cold ablation'. This type of processing is advantageous, because the reflective strain sensitive structure inscribed in the fiber cores may fade at higher temperatures, reducing the sensitivity of the reflective strain sensitive structure.

Optionally, the method may further comprise, after laser ablation, in particular after femtosecond laser ablation, laser processing the surface of the processed area by a longer time scale laser pulse at lower fluence such that the surface is not ablated, but a thin surface layer on the cladding is melted and forms a smooth outer surface of the fiber.

Using this combination of different laser processing types allows for a smooth ablation surface of the thinned part, which improves the mechanical properties (e.g. strain at break) of the thinned tip section.

Furthermore, conventional xyz manipulation stages of the laser target allow easy processing for reduction of the fiber diameter along at least one axis, such that the minimum bending radius orthogonal to that axis is reduced.

In other configurations of the method, the post-processing of the distal tip section may include milling or grinding the cladding of the tip section to reduce the width of the tip section along at least one axis.

In still other configurations of the method, the distal tip section of the optical fiber may be processed by hot-forming or drawing. In this case the reflective strain sensitive structure, e.g. one or more fiber Bragg gratings, must be (re-)written in the tip section which was affected by the high-temperature processing.

Alternatively, the tip section may be made as a separate part and connected to the main section of the optical fiber.

When the tip section is made from a separate piece of optical fiber and connected to the main section of the optical fiber, it is advantageous that the tip section may be provided in advance with a desired shaped cross section, i.e. a reduced width along at least one axis. In this way, a set of distal tip sections may be held in stock. The tip section may be separately processed by any method including for example laser processing or hot-forming or cutting from a longer optical fiber that was differently drawn than the main section. The distal tip section may be connected to the main section by splicing, wherein the common fiber cores of the main section and the distal tip section have to align at the splice.

In an embodiment of the elongated device according to the invention, a ribbon may be arranged along an inner wall of the terminal section of the shell, the ribbon having a thickness in direction of a thickness axis and a width in direction of a width axis, the thickness being smaller than the width, and wherein the multicore optical fiber according to the invention is arranged with respect to the ribbon such that said second axis is parallel to the thickness axis.

In this embodiment, the thinned axis of the multicore optical fiber coincides with the thin axis of the ribbon that provides mechanical stability in the distal tip of the elongated device. The difference in bending resistance of the thick and thin axes of the ribbon will induce a preferential bending direction of the device tip, such that the optical fiber is only bent around the thin dimension. For example, a ribbon with a width dimension that is 3 times larger than the thickness dimension, the resistance to bending in the width direction is 81 times higher than in the thickness direction so that the optical fiber is also safer from accidentally being bent in the wrong direction, i.e. a direction coinciding with the thicker dimension of the optical fiber. In this embodiment, it is advantageous if the distal tip portion of the optical fiber is not free to rotate in the lumen of the elongated device, as the shell of the elongated device has a preferential bending direction due to the ribbon. Thus, the distal tip portion of the optical fiber may be fixed in this embodiment.

### BRIEF DESCRIPTION OF THE DRAWINGS

These and other aspects of the invention will be apparent from and elucidated with reference to the embodiment(s) described hereinafter. In the following drawings:
Fig. 1 shows a piece of length of an example of a multicore optical fiber;
Fig. 2 shows a side view according to an arrow II in Fig. 3 of an embodiment of a multicore optical fiber, wherein fiber cores of the multicore optical fiber are omitted in Fig. 2;
Fig. 3 shows a front view of the multicore optical fiber in Fig. 2, wherein fiber cores of the multicore optical fiber are shown;
Fig. 4 shows a side view according to an arrow IV in Fig. 5 of an embodiment of a multicore optical fiber, wherein fiber cores of the multicore optical fiber are not shown in Fig. 4;
Fig. 5 shows a front view of the multicore optical fiber in Fig. 4; and
Fig. 6 shows a side view of an embodiment of an elongated device comprising a multicore optical fiber according to an embodiment.

### DETAILED DESCRIPTION OF THE INVENTION

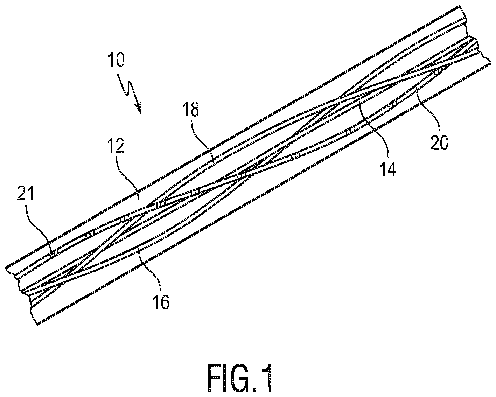

Fig. 1 shows a piece of length of a multicore optical fiber 10 configured for use in FORS technology. The multicore optical fiber 10 comprises a cladding 12 and a plurality of fiber cores 14, 16, 18, 20. The fiber core 14 is a central core arranged on and along the center axis of the optical fiber 10, and fiber cores 16, 18, 20 are outer fiber cores arranged radially apart from the center core 14. The outer cores 16, 18, 20 are helically wound around the central fiber core 14. The outer cores 16, 18, 20 are angularly spaced with respect to one another around the longitudinal center axis of the optical fiber 10. The angular spacing between neighboring fiber cores typically is 120° for a number of three outer fiber cores. The multicore optical fiber 10 may have less than four fiber cores, or more than four fiber cores. For example, the multicore optical fiber 10 may comprise seven fiber cores, with three radially outer fiber cores, one central fiber core, and three other fiber cores in an intermediate radial position between the three outer cores and the central core.

Each of the fiber cores 14, 16, 18, 20 comprises a reflective strain sensitive structure 21 exemplarily shown for fiber core 20 in Fig. 1. The reflective strain sensitive structure may comprise one or more fiber Bragg gratings (FBGs). The reflective strain sensitive structure 21, e.g. the FBG or FBGs, is responsive to strain in the optical fiber 10. The multicore optical fiber 10 can sense strain, which may be axial strain, bending strain or torsional strain, such that 3D shape reconstruction of the multicore optical fiber 10 can be performed with an optical interrogator system as known in the art. When the multicore optical fiber 10 is used in an elongated device, like a guidewire or catheter, it is thus possible to provide a 3D shape reconstruction of the elongated device.

Typical multicore optical fibers with an outer cladding diameter in a range of e.g. 100 µm - 125 µm have been proven to provide good strain sensitivity and, therefore, are very suitable for the FORS technology. In medical interventional applications, such as peripheral procedures, such as treatment of peripheral artery disease, Fiber Optic RealShape (FORS) enabled elongated devices equipped with a multicore optical fiber, especially FORS enabled guidewires, are beneficial for reducing radiation dose. However, in these types of procedures, vessels are very small in diameter, and often the trajectory of navigation is very tortuous (i.e. with many bends), and potentially many bifurcations must be accessed to get to the target location, often involving acute angles through which the elongated device and thus the multicore optical fiber must travel. Moreover, these smaller vessels are much more fragile and may be damaged by the tip of the elongated device. Especially in PAD, angioplasty techniques require crossing a narrowed or occluded section in a blood vessel. For more severe occlusions, this typically requires force and sometimes the tip of the wire prolapses, i.e. the wire loops and the tip bends back on itself, a phenomenon also called 'knuckling' which causes very tight bending in the tip of the device, e.g. with a bending radius < 1 mm.

Wang, Jian-Bo et al. "An Effective Guidewire Looping Technique for the Recanalization of Occlusive Segments of Infrapopliteal Vessels", Korean Journal of Radiology 11 (2010), pages 441-448, describe such a transluminal angioplasty technique. As described there, a hydrophilic guidewire is inserted into a proximal branch of the vessel and then a 'U' shape is made by rotating and advancing the guidewire continually. The looped guidewire is then advanced into and over occlusive segments of the vessel, whereafter a catheter is crossed over the occlusive segment along the guidewire. The guidewire described there is not equipped with an optical fiber.

When the elongated device, like a guidewire, includes an optical fiber extending up to the distal tip of the elongated device, and when the optical fiber is subject to a loop formation as described above, the curvature of the optical fiber may become too high so that shape sensing is impeded, or, when the curvature of the optical fiber becomes even higher than the maximum curvature that the glass fiber can withstand, this results in failure of the glass material of the optical fiber, i.e. the optical fiber may break or shatter.

These technical problems are solved by a new design of a multicore optical fiber embodiments of which will be described below.

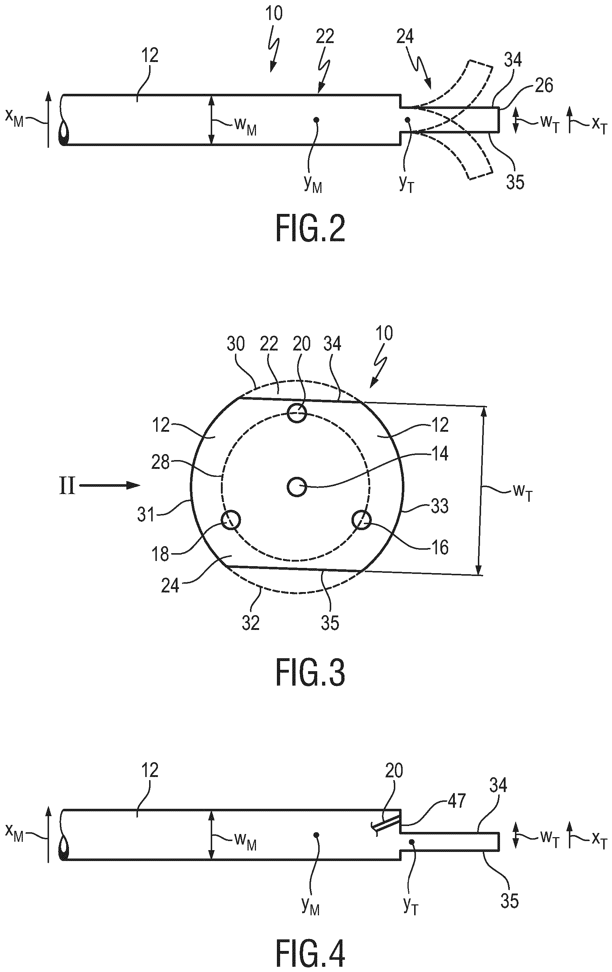

Figs. 2 and 3 show an embodiment of a multicore optical fiber, wherein elements in Figs. 2 and 3, which are identical, similar or comparable with elements in Fig. 1, are labeled with the same reference numerals as in Fig. 1.

Figs. 2 and 3 show a multicore optical fiber 10 comprising a cladding 12 and a plurality of fiber cores 14, 16, 18, 20 embedded in the cladding 12. The fiber cores 14, 16, 18, 20 are not shown in Fig. 2 for the sake of simplicity. Fiber core 14 is a central core longitudinally extending along a center axis of the cladding 12, and fiber cores 16, 18, 20 are outer cores arranged in a radial distance from the center core 14. The fiber cores 14, 16, 18, 20 are equipped with reflective strain sensitive structures responsive to strain in the optical fiber 10 as described above with reference to Fig. 1. The reflective strain sensitive structures may be fiber Bragg gratings.

The cladding 12 comprises along a length of the optical fiber 10 a proximal main section 22 and a distal tip section arranged distally from the main section 22. The distal tip section 24 has a distal end 26. The term "distal" section denotes the section of the fiber 10 which in use is the leading section of the fiber when the fiber is advanced (together with an instrument equipped with the optical fiber), e.g. in a medical application. The term "proximal" section is the trailing section of the optical fiber, accordingly.

The main section 22 may have a configuration as shown in Fig. 1. The main section 22 has a width W_{M} along an axis X_{M} transverse to the longitudinal axis of the optical fiber 10. The term 'width' used in the present disclosure in the context of the main section 22 is to be understood as the diameter of the main section 22, i.e. is used herein as a synonym for 'diameter'. Further, the main section 22 has a first number of fiber cores, in the present embodiment four fiber cores 14, 16, 18, 20. In Fig. 3, the circle 28 shall symbolize the helically wound arrangement of the fiber cores 16, 18, 20, i.e. the outer cores, e.g. as shown in Fig. 1.

The distal tip section 24 which may be exposed to high bending curvatures, i.e. small bending radii, during use in interventional procedures, such as peripheral procedures, such as treatment of peripheral artery disease, has a width W_{T} along an axis X_{T} which is parallel to the axis X_{M}. The term 'width' used in the present disclosure in the context of the distal tip section 24 is to be understood as the thickness of the distal tip section 24, i.e. is used herein as a synonym for 'thickness'. The size dimension of the thickness W_{T} is reduced with respect to the size dimension of the diameter W_{M} of the main section 22 such that the bending stiffness of the tip section 24 about a bending axis Y_{T} orthogonal to the axis X_{T} is lower than the bending stiffness of the main section 22 about a bending axis Y_{M} which is parallel to the bending axis Y_{T} and orthogonal to the axis X_{M}. The bending axes Y_{T} and Y_{M} are perpendicular to the plane of drawing in Fig. 2.

In the embodiment of Fig. 2, the width or thickness of the tip section 24 is reduced along one axis only, namely the axis X_{T}, while the thickness of the tip section 24 along an axis perpendicular to the axis X_{T} may be the same as the diameter of the main section 22 along this axis.

In Fig. 3, lines 31, 33 and broken lines 30, 32 show the outer circumference or surface of the main section 22, and the lines 31, 33 and 34, 35 show the outer circumference or surface of the tip section 24.

It is also possible that the tip section 24 has a reduced width or thickness along more than one axis, for example the width of the tip section 24 may also be reduced along an axis perpendicular to the axis X_{T}.

In the present embodiment, the tip section 24 has two flattened lateral surfaces corresponding to the lines 34, 35 in Fig. 3. The flattened surfaces 34, 35 are opposite to one another with respect to the longitudinal center axis of the optical fiber 10, and, in particular, may be parallel to one another.

In the embodiment of Figs. 2 and 3, the tip section 24 has a preferential bending direction, namely about the bending axis Y_{T}, while the stiffness of the tip section 24 about a bending axis perpendicular to the bending axis Y_{T} is more or less the same as the higher bending stiffness of the main section 22. In Fig. 2, bending of the tip section 24 about the bending axis Y_{T} in two opposite bending directions is illustrated by broken lines.

Further, in this embodiment, the tip section 24 has the same number of fiber cores as the main section 22, i.e. all fiber cores 14, 16, 18, 20 extend into the distal tip section 24, preferably up to the distal end 26 of the tip section 24.

In the embodiment of Figs. 2 and 3, the distal tip section 24 is flattened such that it is symmetrical with respect to the longitudinal center axis of the cladding 12.

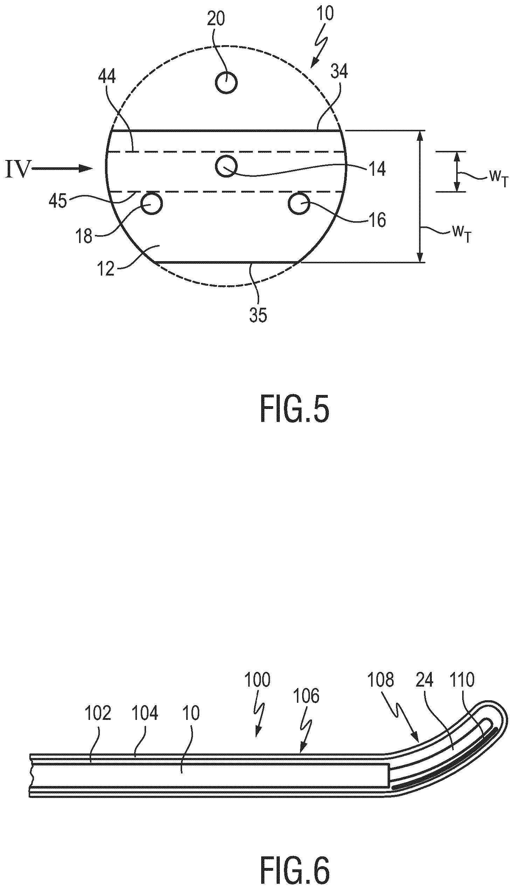

Figs. 4 and 5 show another embodiment of an optical fiber 10, wherein elements of the optical fiber 10 in Figs. 4 and 5 which are identical, similar or comparable with elements of the optical fiber 10 in Figs. 1, 2 and 3 are labeled with the same reference numerals as in Figs. 1, 2 and 3.

In the following, only the differences between the embodiment in Figs. 4 and 5 with respect to the embodiment in Figs. 2 and 3 will be described.

The tip section 24 of the optical fiber 10 in Figs. 4 and 5 has a thickness W_{T} along the axis X_{T} which is further reduced in comparison with the thickness W_{T} of the distal tip section 24 of the optical fiber 10 in Figs. 2 and 3. Thus, the flattened surfaces 34 and 35 of the distal tip section 24 have a smaller distance from one another than in the distal tip section 24 of the optical fiber 10 in Figs. 2 and 3. Further, as exemplified in Figs. 4 and 5, the distal tip section 24 is asymmetrical with respect to the longitudinal center axis of the cladding 12 of the main section.

Furthermore, the number of fiber cores in the tip section 24 is reduced in comparison to the number of fiber cores in the main section 22. In the present embodiment, at least one fiber core, and exemplarily shown for fiber core 20 in Fig. 4, terminates at or proximally from the start of the tip section 24, while fiber cores 14, 16, 18 extend into the distal tip section 24 and terminate at the distal end 26 of the tip section 24.

Fig. 5 shows with broken lines 45 that the width or thickness W_{T} of the tip section 24 may be, in other embodiments, further reduced such that also the fiber cores 16 and 18 terminate at or proximally from the start of the tip section 24, while only the central fiber core 14 extends into the distal tip section 24 and terminates at the distal end 26 of the tip section 24.

Fiber core(s) terminating at the distal end of the main section 22, like fiber cores 20 and/or 16, 18, preferentially terminate(s) under an angle with respect to the longitudinal axis of the optical fiber 10 to reduce reflections at the front surface(s) of the terminated fiber core(s) back into the fiber core(s), as exemplarily shown for fiber core 20 in Fig. 4.

In the present embodiment, the fiber cores 14, 16 and 18 in the distal tip section 24 are arranged eccentrically with respect to a neutral bending plane of the cladding of the tip section 24. The neutral bending plane of the tip section 24 is the central plane in the mid between the surfaces 34 and 35 and parallel thereto.

In a further embodiment, the width or thickness W_{T} of the tip section 24 may be further reduced as illustrated by surfaces 44, 45 such that only one fiber core, in particular the central fiber core 14 is present in the tip section 24, preferably wherein the central fiber core 14 is arranged eccentrically with respect to the neutral bending plane of the cladding 12 of the tip section 24, wherein the neutral bending plane is the middle plane between and parallel to the surfaces 44 and 45 of the tip section 24. This means that even the central core 14 which, in the main section 22, is not sensitive to bending deformation, becomes sensitive to bending deformation in the tip section 24.

It is also possible to reduce the width or thickness W_{T} of the distal tip section 24 such that the surfaces 35 and 45 form the contour of the distal tip section 24. In this case, the fiber cores 16, 18 extend into the distal tip section and are arranged eccentrically with respect to the neutral bending plane of the tip section 24, wherein the neutral bending plane then is the middle plane between and parallel to the surfaces 45 and 35 of the distal tip section 24.

The bending stiffness of the tip section 24with width or thickness W_{T} may be in a range from 0.05 Nmm² - 0.2 Nmm². For example, a typical 100 µm diameter glass optical fiber has a bending stiffness of 0.34 Nmm². A reduction of the thickness along axis X_{T} in the tip section 24 will decrease the bending stiffness of the tip section 24. For example, a thickness or width reduction of 20 µm cladding resulting in a 80 µm width W_{T} will reduce the bending stiffness to 0.18 Nmm².

Preferably, the tip section 24 in all embodiments described above has a length in a range from 1 to 5 cm.

In the following, embodiments of a method for producing the multicore optical fiber 10 will be described.

A standard or conventional multicore optical fiber is provided. The reduced width W_{T} of the tip section 24 may be achieved by post-processing the distal tip section of the conventional multicore optical fiber by laser ablation of the cladding 12 in the distal tip section 24 such that the cladding material of the cladding 12 is removed in the distal tip section 24.

Laser ablation may be performed as femtosecond laser ablation. Femtosecond laser ablation is a technology allowing highly precise removal of material while minimizing the heat affected zone around the processed area, where the material is removed, which is typically less than a few micrometers. Hence, this form of laser ablation is also referred to as 'cold ablation'. For a multicore optical fiber like the optical fibers 10 in Figs. 2 to 5 'cold ablation' is advantageous, because the reflective strain sensitive structure, in particular in form of fiber Bragg gratings inscribed in the optical fiber 10 may fade at higher temperatures, reducing the sensitivity of the fiber Bragg grating(s).

Optionally, the surface of the processed area, e.g. the surfaces 34, 35 or 44, 45 may be further laser processed by a longer time scale laser pulse at lower fluence such that the surface is not ablated, but a thin surface layer of the cladding 12 is melted and forms a smooth outer surface of the optical fiber 10.

Using this combination of different laser processing types allows for a smooth ablation surface on the thinned distal tip section 24, which improves the mechanical properties (e.g. strain at break) of the thinned distal tip section.

Conventional xyz manipulation stages of the laser target allow easy processing for reduction of the width of the optical fiber 10 along one axis, such that the minimum bending radius orthogonal to that axis is reduced as in case of the embodiments in Figs. 2-5.

Alternatively, other processing technologies may be used to shape the distal tip section 24, such as milling or grinding, or higher temperature processing steps such as hot-forming or drawing, after which the reflective strain sensitive structure, e.g. fiber Bragg gratings, must be (re-)written in a distal tip section of the optical fiber 10 that was affected by the high temperature processing, to allow strain sensing with FBGs.

Alternatively, the distal tip section 24 may be produced from a separate optical fiber or piece of optical fiber with a cross section of desired shape, i.e. a cross section with reduced width W_{T}. The distal tip section 24 may then be spliced to a standard optical fiber to form the main section 22 of the optical fiber 10. In particular, a single optical fiber with an eccentric core to allow derivation of bending information, preferably in a preferential direction of bending may be spliced to the main section 22.

The distal tip section 24 may be separately processed by any method including for example laser processing or hot-forming or cutting from a long optical fiber that was differently drawn, as long as the fiber core(s) in the distal tip section 24 align(s) with the corresponding fiber core(s) in the main section 22 of the optical fiber 10.

In the embodiments described above, the outer fiber cores 16, 18, 20 preferentially are twisted in a helix. In this case, the effective length of the remaining outer fiber cores like fiber cores 16, 18 (Fig. 5) in the distal tip section will span less than 1 full twist length, limiting the length of the distal tip section 24 depending on the twist in the original optical fiber.

Alternatively, the separate fiber sensor section with the reduced number of cores may be spliced to the multicore fiber main section 22, wherein the fiber cores in the distal tip section may or may not be twisted.

Fig. 6 shows an embodiment of an elongated device for medical application labeled with general reference numeral 100. The elongated device 100 comprises a longitudinal lumen 102 and a shell 104 surrounding the lumen. The shell has a main section 106 and a distal terminal section 108. The terminal section 108 has a bending stiffness which is lower than the bending stiffness of the main section 106 of the shell 104. A multicore optical fiber 10 like one of those shown in Figs. 2-5 and described above is arranged in the lumen 102, wherein the tip section 24 of the optical fiber 10 is arranged in the distal terminal section 108 of the shell 104.

In the embodiments of Figs. 2 to 5, the distal tip section 24 has a preferential bending direction, namely in the plane of drawing in Figs. 2 and 4. In order to further support this preferential bending direction, a ribbon 110 may be arranged along an inner wall of the terminal section 108 of the shell 104, which has a thickness in direction of a thickness axis (parallel to the plane of drawing in Fig. 6) and a width in direction of a width axis (perpendicular to the thickness axis) of the ribbon 110, wherein the thickness of the element 10 is smaller than its width, and wherein the multicore optical fiber 10 is arranged with respect to the ribbon such that the axis X_{T} (Fig. 2, Fig. 4) is parallel to the thickness axis of the element 110, or in other words, the distal tip section 24 faces the wider side of the element 110. The difference in bending resistance of the thickness direction and width direction of the ribbon 110 will induce a preferential bending direction of the distal terminal section 108 of the device 100 about the width axis of the element 110 such that the distal tip section 24 of the optical fiber 10 is only bent around the thin dimension, e.g. orthogonal to the thinnest dimension of the distal tip section 24. For example, a ribbon with a width dimension that is three times larger than the thickness dimension, the resistance to bending in the width direction is 81 times higher than in the thickness direction so that the distal tip section 24 of the optical fiber 10 is safer from accidentally being bent in the wrong direction (i.e. a direction coinciding with the thicker dimension of the distal tip section 24 (perpendicular to the plane of drawing in Figs. 2 and 4)).

While it is preferred, if the distal tip section 24 is free to rotate within the lumen 102 of the elongated device 100, such a rotatability of the distal tip section 24 is not needed when the ribbon 110 is provided which by itself provides a preferential bending direction. In contrast, it is advantageous in this case when the distal tip section 24 of the optical fiber 10 is fixed in the lumen 102 of the device 100.

In the following, example values and ranges of preferable bending stiffnesses of the distal tip section 24 and related values and ranges of the thickness W_{T} of the distal tip section 24 for certain interventional applications will be provided.

In first order approximation, the bending stiffness of the distal tip section of a guidewire in Nmm² is about 1/10th of the tip load in grams (tip load is the numerical conversion of the physician's feeling of "softer/stiffer" of the guidewire tip and is calculated by its gram value). In general, the tip loads for coronary navigation (non-crossing) guidewires range from 0.5 grams up to 2-3 grams: For coronary (frontline) navigation: 0.5-1 gram, thus bending stiffness is in a range of 0.05-0.1 Nmm²; for coronary tortuous anatomy navigation: 1.5-3 grams, thus bending stiffness is in a range of 0.15-0.3 Nmm².

In general, the tip load is related to the guidewire diameter, wherein, for example, 0.035" diameter guidewires are much stiffer than 0.018" diameter guidewires, which in turn are stiffer than 0.014" diameter guidewires.

For instance, 0.018" diameter navigation guidewires have a typical tip load of 1-4 grams (0.1 - 0.4 Nmm² bending stiffness), which are suitable for a variety of peripheral procedures (e.g. peripheral angiography, angioplasty, thrombectomy, atherectomy, embolization).

Considering the total bending stiffness of the tip of the guidewire is 0.05 - 0.4 Nmm², the bending stiffness of the optical fiber arranged in the guidewire must be less than that of the tip of the guidewire. Typically, the bending stiffness of the optical fiber is not more than half of the bending stiffness of the guidewire tip.

The bending stiffness (EI) of circular cross section glass optical fibers are as follows:

| Fiber diameter (microns) | Bendingstiffness [Nmm2] |
|---|---|
| 125 | 0.84 |
| 100 | 0.34 |
| 80 | 0.14 |

For 125 and 100 micron diameter optical fibers the bending stiffness of the optical fiber is already almost equal to (for the 100 micron diameter) or even higher (for 125 micron) than the ideal bending stiffness of the guidewire tip consisting of the optical fiber and a housing (typically a coil or other flexible structure type).

For several reasons, including geometric constraints, the coil is typically of equal bending stiffness as the optical fiber inside it, such that the range of stiffnesses required for the optical fiber to achieve a competitive tip load with respect to other guidewires without optical fiber is about half of the total tip bending stiffness of the guidewire, i.e. the optical fiber bending stiffness is in the range of 0.025-0.02 Nmm². In the table below the bending stiffness in preferential bending directions of thinned distal tip sections, like distal tip section 24, with thickness W_{T} in one axis, starting from typical 125 or 100 micron diameter optical fibers is given:

| W_{T} of distal tip section [µm] | Bending stiffness when thinned from 125 µm [Nmm²] | Bending stiffness when thinned from 100 µm [Nmm²] |
|---|---|---|
| 75 | 0.271 | 0.209 |
| 60 | 0.145 | 0.113 |
| 50 | 0.088 | 0.069 |
| 40 | 0.046 | 0.037 |
| 35 | 0.031 | 0.025 |

Results in the table above show that the clinically required range of about 0.025 - 0.02 Nmm² is achievable using the principle according to the invention of thinning a distal tip section, like distal tip section 24, of conventionally sized optical fibers.

Another important reason to reduce the diameter of the optical fiber is the tightest bend that the optical fiber can survive without breaking. There are multiple reasons that the guidewire may experience a tight bend.

A typical value for strength of the fiber under dynamic bending conditions for the duration of typical clinical procedure times (up to a few hours) is around 3 GPa (see Fig. 8 of M. Matthewson, Fiber Optics Reliability and Testing SPIE Critical Reviews of Optical Science and Technology Volume CR50, pp. 3-31, 1993).

The bending stress is proportional to the ratio of the optical fiber thickness (or W_{T} in the distal tip section 24) and the bending radius. So a smaller W_{T} will allow a smaller bending radius.

The 3 GPa upper stress limit will translate to a bending radius of about 2.9mm for a standard optical fiber of 125 microns, which is not enough to make a 180 degree bend when prolapsing inside a typical peripheral vessel having about 2 to 3mm diameter.

Thinning will allow a bending radius of less than 1.5mm such that a prolapse inside a small vessel below 3mm will not damage the optical fiber. In the following table, critical bending radii R for a range of thicknesses W_{T} of the distal tip section 24 of the optical fiber are given:

| W_{T} (microns) | critical R [mm] |
|---|---|
| 125 | 1.5 |
| 100 | 1.2 |
| 60 | 0.7 |
| 45 | 0.5 |

The desired thinning thickness W_{T} at the distal tip section depends (linearly) on the minimum bending radius that needs to be achieved in the procedure (typically down to 2-3 mm in peripheral vessels).

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality. A single element or other unit may fulfill the functions of several items recited in the claims. The mere fact that certain measures are recited in mutually different dependent claims does not indicate that a combination of these measures cannot be used to advantage.

Any reference signs in the claims should not be construed as limiting the scope.

## Claims

1. Multicore optical fiber configured for optical shape sensing, comprising a cladding (12) and a plurality of fiber cores (14, 16, 18, 20) embedded in the cladding (12), the fiber cores (14, 16, 18, 20) each have a reflective strain sensitive structure (21) responsive to strain in the optical fiber (10), wherein the cladding (12) comprises, along a length of the optical fiber (10), a main section (22) and a distal tip section (24) arranged distally from the main section (22), the main section (22) having a first width along a first axis transverse to a longitudinal axis of the optical fiber (10) and a first number of fiber cores, and the tip section (24) having a second width along a second axis parallel to said first axis and a second number of fiber cores, wherein the second width is reduced with respect to the first width such that a bending stiffness of the tip section (24) about a bending axis orthogonal to said second axis is lower than the bending stiffness of the main section (22) about a bending axis orthogonal to said first axis.

2. Multicore optical fiber of claim 1, wherein the cladding (12) of the tip section (24) has at least one flattened lateral surface (34, 35, 44, 45) along the length of the tip section (24).

3. Multicore optical fiber of claim 2, wherein the cladding (12) of the tip section (24) has a first flattened lateral surface (34, 44) and a second flattened lateral surface (35, 45) along the length of the tip section (24) , wherein the first flattened lateral surface (34, 44) is opposite the second flattened lateral surface (35, 45).

4. Multicore optical fiber of any one of claims 1 to 3, wherein the second number of fiber cores (14, 16, 18, 20) is equal to the first number of fiber cores (14, 16, 18, 20).

5. Multicore optical fiber of any one of claims 1 to 3, wherein the second number of fiber cores (14, 16, 18) is equal to or larger than one, but smaller than the first number of fiber cores (14, 16, 18, 20).

6. Multicore optical fiber of claim 5, wherein fiber cores (16, 18, 20) of a first subset of the fiber cores (14, 16, 18, 20) of the main section (22) terminate at or proximally from the beginning of the tip section (24), and fiber cores (14, 16, 18) of a second subset of the fiber cores (14, 16, 18, 20) of the main section (22) extend into the tip section (24).

7. Multicore optical fiber of claim 6, wherein the fiber cores (16, 18, 20) of the first subset of the fiber cores (14, 16, 18, 20) of the main section (22) terminate under an angle such that reflections of light are reduced.

8. Multicore optical fiber of any one of claims 1 to 7, wherein one or more of the fiber cores (14, 16, 18) of the tip section (24) are arranged eccentrically with respect to a neutral bending plane of the cladding (12).

9. Multicore optical fiber of any one of claims 1 to 8, wherein the fiber cores (14, 16, 18, 20) of the main section (22) comprise a central core (14) arranged centrically with respect to a neutral bending plane of the cladding (12) of the main section (22), wherein a distal portion of the central core (14) extends into the tip section (24), and wherein the distal portion of the central core (14) is arranged eccentrically with respect to a neutral bending plane of the cladding (12) of the tip section (24).

10. Multicore optical fiber of any one of claims 1 to 9, wherein the tip section (24) has a bending stiffness about the bending axis in a range from 0.05 Nmm² - 0.2 Nmm².

11. Multicore optical fiber of any one of claims 1 to 10, wherein the tip section (24) has a length in a range from 1 to 5 cm.

12. Multicore optical fiber of any one of claims 1 to 11, wherein the main section (22) and the tip section (24) are made from a single monolithic fiber.

13. Multicore optical fiber of any one of claims 1 to 11, wherein the tip section (24) is made as a separate part and connected to the main section (22) of the optical fiber (10).

14. Elongated device for medical interventional application, comprising:
a main section (106) and a distal terminal section (108) together defining a lumen (102) along a length of the elongated device (100); and
a multicore optical fiber (10) according to any one of claims 1 to 13 which is arranged in the lumen (102), wherein the tip section (24) of the optical fiber (10) is arranged in the distal terminal section (108) of the elongated device (100).

15. Elongated device of claim 14, further comprising a ribbon (110) arranged along an inner wall of the terminal section (108), the ribbon element (110) having a thickness in direction of a thickness axis and a width in direction of a width axis, the thickness being smaller than the width, and wherein the multicore optical fiber is arranged with respect to the ribbon (110) such that said second axis is parallel to the thickness axis.
